# DEMANDE DE BREVET EUROPEEN

(11) **EP 1 462 086 A1**
(43) Date de publication de la demande: **29.09.2004**
(21) Numéro de dépôt: 04290562.0
(22) Date de dépôt: 02.03.2004
(51) Int. Cl.: A61K 7/06, A61K 7/50

(54) **Utilisation d'un acide carboxylique particulier ou ses sels, comme agents de conditionnement des matières kératiniques**

(30) Priorité: 25.03.2003 FR 0303641; 25.03.2003 FR 0303637; 28.03.2003 FR 0303879
(71) Demandeur: L'OREAL, 75008 Paris (FR)
(72) Inventeur: Müller, Rainer, 76344 Leopoldshafen (DE)
(74) Mandataire: Le Blainvaux Bellegarde, Françoise

(57) **Abrégé**

L'invention concerne l'utilisation d'au moins un acide carboxylique ou ses sels répondant à la formule générale (I) ou (II) suivantes :

R¹-(CHOH)₄-CO₂X (I)

R²-N-(CH(R')COOX)₂ (II)

dans lesquelles :
- R¹ représente un groupe CH₂OH ou CO₂X,
- X représente un atome d'hydrogène ou un cation monovalent ou divalent issu d'un métal de transition, d'un métal alcalin ou alcalinoterreux, d'une amine organique ou d'un ion ammonium,
- R² représente un atome d'hydrogène ou un groupe -CH(COOX)-(CH₂)₂COOX, -CH₂CH₂OH, -CH(CH₃)-COOX ou -(CH₂)₂-N(COR")-CH₂-COOX ;
- R" représente un groupe alkyle, linéaire ou ramifié, comportant de 1 à 30 atomes de carbone ou cyclique comportant de 3 à 30 atomes de carbone ;
- R' représente un groupe -CH₂-COOX lorsque R² représente un atome d'hydrogène, tandis que R' représente un atome d'hydrogène lorsque R² est différent d'un atome d'hydrogène,
comme agent de conditionnement des matières kératiniques en particulier les fibres kératiniques et plus particulièrement les cheveux dans et pour la préparation de compositions cosmétiques, notamment capillaires.

## Description

La présente invention concerne l'utilisation d'un acide carboxylique particulier ou ses sels, comme agents de conditionnement des matières kératiniques dans et pour la préparation de compositions cosmétiques comme agents de conditionnement des matières kératiniques en particulier les fibres kératiniques et plus particulièrement les cheveux.

Les compositions cosmétiques contiennent généralement un agent complexant destiné à complexer les cations métalliques susceptibles de se trouver à l'état de traces dans ces compositions, ainsi que ceux pouvant être présents sur les cheveux et provenant de l'air ambiant, de l'eau avec laquelle ces derniers ont été lavés ou encore des shampoings ou autres produits capillaires avec lesquels ils ont été traités.
Il est, en effet, très important de neutraliser ces cations métalliques, dans la mesure où ils sont susceptibles de catalyser les réactions d'oxydation au niveau des fibres capillaires et ce, de façon non contrôlée, ce qui peut se traduire par des effets indésirables sévères tels qu'une cassure des cheveux ou une brûlure du cuir chevelu.
Actuellement, les agents complexants les plus couramment utilisés dans les compositions oxydantes pour la coloration, la décoloration ou la déformation permanente de fibres kératiniques sont l'acide éthylènediamine tétraacétique (EDTA) et ses dérivés comme l'acide diéthylènetriamine pentaacétique (DPTA), généralement dans des proportions pondérales de l'ordre de 0,1 à 1%. Ces agents complexants ne sont pas suffisamment biodégradables.

Par ailleurs, la Demanderesse a constaté que l'EDTA et ses dérivés présentent, dans ce type de compositions, des propriétés complexantes insuffisantes. Ces constatations, qui sont corroborées par les résultats obtenus par d'autres équipes de recherche, justifient de trouver de nouveaux agents complexants.

La demanderesse a découvert de façon surprenante que des agents complexants particuliers, utilisés dans des compositions cosmétiques notamment capillaires, confèrent aux cheveux un toucher particulièrement doux et facilitent leur démêlage. Ils peuvent donc être utilisés comme agents de conditionnement des matières kératiniques en particulier les fibres kératiniques et plus particulièrement les cheveux.

Un objet de l'invention concerne donc l'utilisation d'au moins un acide polycarboxylique particulier ou ses sels, comme agent de conditionnement des matières kératiniques en particulier les fibres kératiniques et plus particulièrement les cheveux dans et pour la préparation de compositions cosmétiques, notamment capillaires.

D'autres objets apparaîtront à la lumière de la description et des exemples qui suivent.

Dans le cadre de la présente demande, on entend par agent de conditionnement un agent ayant pour fonction l'amélioration des propriétés cosmétiques des cheveux, en particulier la douceur, le démêlage, le toucher, la brillance, l'électricité statique.

Les acides carboxyliques particuliers ou ses sels sont choisis parmi les acides de formule générale (I) ou (II) suivantes :

R¹-(CHOH)₄-CO₂X (I)

R²-N-(CH(R')COOX)₂ (II)

dans lesquelles :
- R¹ représente un groupe CH₂OH ou CO₂X,
- X représente un atome d'hydrogène ou un cation monovalent ou divalent issu d'un métal de transition, d'un métal alcalin ou alcalinoterreux, d'une amine organique ou d'un ion ammonium,
- R² représente un atome d'hydrogène ou un groupe -CH(COOX)-(CH₂)₂COOX, -CH₂CH₂OH, -CH(CH₃)-COOX ou -(CH₂)₂-N(COR")-CH₂-COOX;
- R" représente un groupe alkyle, linéaire ou ramifié, comportant de 1 à 30 atomes de carbone ou cyclique comportant de 3 à 30 atomes de carbone ;
- R' représente un groupe -CH₂-COOX lorsque R² représente un atome d'hydrogène, tandis que R' représente un atome d'hydrogène lorsque R² est différent d'un atome d'hydrogène.

Ainsi, les agents complexants de formule (I) utilisés dans le cadre de l'invention correspondent à des acides hydroxycarboxyliques et aux carboxylates correspondants.

La formule (I) comprenant 4 groupes d'atomes H-C-OH chiraux, et même 5 lorsque R représente un groupe CH₂OH, il va de soi que cette formule englobe tous les énantiomères et tous les diastéréoisomères des composés susceptibles de répondre à cette formule.
Conformément à l'invention, lorsque le ou les composés de formule (I) sont des carboxylates, alors le cation monovalent ou divalent est, de préférence, choisi dans le groupe constitué par les cations de métaux alcalins, les cations de métaux alcalino-terreux, les cations divalents de métaux de transition et les cations monovalents issus d'amines organiques ou d'ammonium.

A titre d'exemples de cations de métaux alcalins, on peut notamment citer le sodium (Na⁺) et le potassium (K⁺), tandis qu'à titre d'exemples de cations de métaux alcalino-terreux, on peut notamment citer le calcium (Ca²⁺) et le magnésium (Mg²⁺).

Au sens de la présente invention, on entend par "métal de transition", un métal comportant une sous-couche d incomplète, plus particulièrement à l'état d'oxydation II, tel que le cobalt (Co²⁺), le fer (Fe²⁺), le manganèse (Mn²⁺), le zinc (Zn²⁺) et le cuivre (Cu²⁺).

En ce qui concerne les sels d'amines organiques, on peut citer les sels d'amine primaire, secondaire ou tertiaire, ou encore d'alcanolamine.
Lesdites amines présentent un ou plusieurs radicaux, identiques ou non, de type alkyle, linéaire ou ramifié en C1 à C20, comprenant éventuellement un hétéroatome comme l'oxygène.

Pour ce qui a trait aux sels d'ammonium quaternaires, ces derniers comprenant trois radicaux, identiques ou non, choisis parmi l'hydrogène, un radical alkyle, linéaire ou ramifié en C1 à C20, comprenant éventuellement un hétéroatome comme l'oxygène.

Lorsque R représente un groupe CH₂OH, alors le ou les composés de formule (I) sont, de préférence, choisis dans le groupe constitué par l'acide gluconique (C₆H₁₂O₇), ses sels de métaux alcalins, ses sels de métaux alcalino-terreux, ses sels de métaux de transition, et leurs mélanges comme, par exemple, des mélanges d'acide gluconique et de gluconate de sodium.

Plus particulièrement, le ou les composés de formule (I) sont alors choisis dans le groupe constitué par l'acide gluconique, le gluconate de sodium (C₆H₁₁O₇Na), le gluconate de potassium (C₆H₁₁O₇K), le gluconate de calcium anhydre (C₁₂H₂₂O₁₄Ca), le gluconate de calcium monohydraté (C₁₂H₂₂O₁₄Ca.H₂O), le boro-gluconate de calcium (C₁₂H₂₂O₁₄Ca.H₂O+H₅BO₅), le gluconate de magnésium (C₁₂H₂₂O₁₄Mg), le gluconate de fer (C₁₂H₂₂O₁₄Fe), le gluconate de manganèse (C₁₂H₂₂O₁₄Mn), le gluconate de zinc (C₁₂H₂₂O₁₄Zn) et le gluconate de cuivre (C₁₂H₂₂O₁₄Cu).

Lorsque R représente un groupe CO₂X, alors le ou les composés de formule (I) sont, de préférence, choisis dans le groupe constitué par l'acide mucique (C₆H₁₀O₈)- encore connu sous le nom d'acide galactarique -, l'acide glucarique (C₆H₁₀O₈), l'acide mannarique (C₆H₁₀O₈), leurs sels de métaux alcalins, leurs sels de métaux alcalino-terreux, leurs sels de métaux de transition, et leurs mélanges comme, par exemple, des mélanges d'acide mucique et de mucate de sodium (C₆H₈O₈Na₂).

De manière particulièrement préférée, le ou les composés de formule (I) sont choisis parmi l'acide gluconique et l'acide mucique.

Ainsi, les acides de formule (II) utilisés dans le cadre de l'invention, correspondent à des composés acides polycarboxyliques et aux carboxylates correspondants.

Plus précisément, ils correspondent à :
- des composés comprenant quatre fonctions acides carboxyliques ou carboxylates, lorsque R² représente un atome d'hydrogène et R' représente un groupe -CH₂-COOX, ou lorsque R² représente le groupe -CH(COOX)-(CH₂)₂-COOX et R' représente un atome d'hydrogène ;
- des composés comprenant trois fonctions acides carboxyliques ou carboxylates, lorsque R² représente le groupe -CH(CH₃)-COOX et R' représente un atome d'hydrogène, ou lorsque R² représente un groupe -(CH₂)₂-N(COR")-CH₂-COOX et R' représente un atome d'hydrogène ; et à
- des composés comprenant deux fonctions acides carboxyliques ou carboxylates, lorsque R² représente le groupe -CH₂CH₂OH et R' représente un atome d'hydrogène.

Le ou les composés de formule (I) sont, de préférence, choisis dans le groupe constitué par l'acide méthylglycine diacétique, l'acide N-lauroyl-N,N',N'-triacétique éthylènediamine, l'acide N,N-dicarboxyméthyl L-glutamique, l'acide iminodisuccinique, leurs sels de métaux alcalins, leurs sels de métaux alcalinoterreux, leurs sels de métaux de transition, leurs sels d'amines organiques, leurs sels d'ammonium, et leurs mélanges.

L'acide méthylglycine diacétique, l'acide 2-hydroxyéthyl iminodiacétique, l'acide N-lauroyl-N,N',N'-triacétique éthylènediamine, l'acide N,N-dicarboxyméthyl L-glutamique, l'acide iminodisuccinique, et leurs sels sont respectivement représentés par les formules (III), (IV), (V),(VI) et (VII) suivantes : 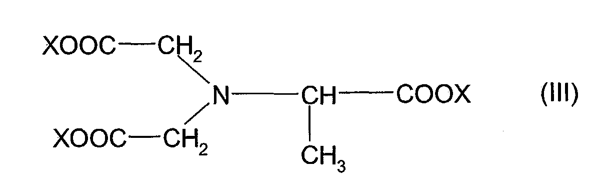 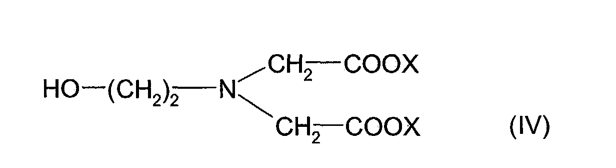 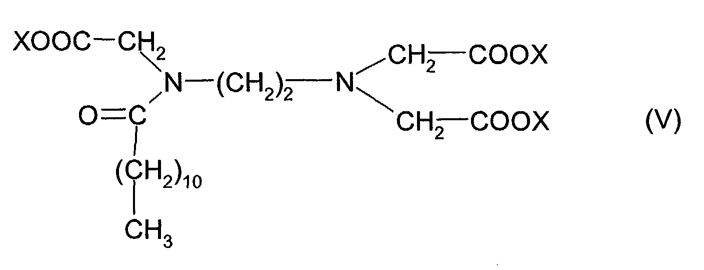 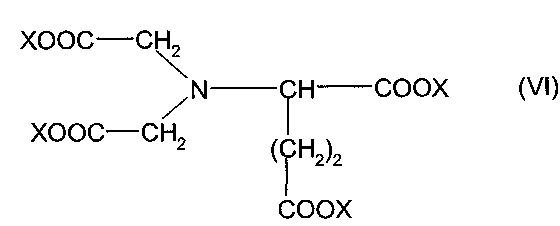 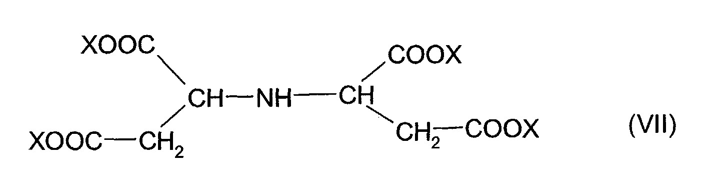 dans lesquelles X est tel que défini précédemment, X correspondant, de préférence, à H ou Na.

Ces composés sont notamment disponibles auprès des sociétés BASF, DOW CHEMICAL, HAMPSHIRE, BAYER et SHOWA DENKO.

Plus particulièrement, on préfère l'acide méthylglycine-diacétique, et ses sels de sodium.

De préférence, le ou les composés de formule (I) ou de formule (II) représentent de 0,001 à 10% en poids et, mieux encore, de 0,001 à 5% en poids du poids total de la composition.

Les compositions cosmétiques, utilisées selon l'invention contiennent un milieu cosmétiquement acceptable, c'est-à-dire un milieu compatible avec la peau, les lèvres, le cuir chevelu, les cils, les yeux, les ongles et/ou les cheveux. Ce milieu cosmétiquement acceptable peut être plus particulièrement constitué d'eau et éventuellement d'un solvant organique physiologiquement acceptable choisi par exemple parmi les alcools inférieurs comportant de 1 à 8 atomes de carbone et en particulier 1 à 6 atomes de carbone, comme l'éthanol, l'isopropanol, le propanol, le butanol ; les polyéthylène glycols ayant de 6 à 80 oxydes d'éthylène ; les polyols comme le propylène glycol, l'isoprène glycol, le butylène glycol, la glycérine, le sorbitol.

Les compositions peuvent se présenter sous forme de lotion aqueuse ou hydroalcoolique, monophasique ou polyphasique, de gel monophasique ou polyphasique, d'émulsion, de crème, de dispersion vésiculaire de lipides ioniques ou non ioniques, lesdites vésicules pouvant alors servir en tant qu'agent d'encapsulation pour des ingrédients actifs lipophiles ou hydrophiles, de mousse, de spray.

En particulier, les compositions pour le soin de la peau selon l'invention peuvent se présenter sous forme de lotion, de gel, d'émulsion, de crème ou de mousse à appliquer sur la peau.

Les compositions capillaires peuvent se présenter sous forme de shampooing, d'après-shampooing à rincer ou non, de compositions pour permanente, défrisage, coloration ou décoloration, ou encore sous forme de compositions à rincer, à appliquer avant ou après une coloration, une permanente ou un défrisage ou encore entre les deux étapes d'une permanente ou d'un défrisage.

Les compositions cosmétiques ou dermatologiques peuvent par ailleurs contenir des additifs cosmétiques conventionnels choisis parmi les corps gras, les solvants organiques, les silicones, les épaississants, les adoucissants, les tensio-actifs, les polymères anioniques, cationiques, non-ioniques ou amphotères, les agents anti-mousses, les agents conditionneurs du cheveu tels que des protéines, des vitamines, les agents traitants (agents anti-chute, antipelliculaires), les colorants, les parfums, les conservateurs, les agents propulseurs.

Parmi les huiles, on peut citer les huiles minérales, animales, végétales ou les huiles de synthèse, et notamment l'huile de vaseline, de paraffine, de ricin, de jojoba, de sésame, ainsi que les huiles et les gommes de silicone et les isoparaffines.

Parmi les cires, on peut citer les cires animales, végétales, minérales ou de synthèse, et notamment les cires d'abeilles, de Candelila, les ozokérites, les cires microcristallines ainsi que les cires et résines de silicone.

Parmi les solvants organiques usuellement utilisés dans les compositions cosmétiques, on peut citer plus précisément les mono-alcools ou polyalcools inférieurs en C₁ à C₆ comme l'éthanol, l'isopropanol, l'éthylèneglycol, le diéthylènegycol, le propylèneglycol, le glycérol.

Les agents épaississants peuvent être choisis notamment parmi l'alginate de sodium, la gomme arabique, les dérivés cellulosiques tels que la méthylcellulose, l'hydroxy méthylcellulose, l'hydroxyéthylcellulose, l'hydroxypropylcellulose, l'hydroxypropylméthyl cellulose, la gomme de guar ou ses dérivés, la gomme de xanthane, les scléroglucanes, les acides polyacryliques réticulés et les polymères associatifs notamment les polymères à chaînes grasses (C6-C30).

Comme agents tensio-actifs et comme polymères , on peut utiliser tous ceux bien connus de l'état de la technique notamment pour leur utilisation dans des compositions capillaires.
Les polymères par exemple cationiques susceptibles d'être utilisés dans le cadre de la présente invention, sont notamment les dérivés d'éther de cellulose quaternaires tels que les produits vendus sous la dénomination « JR 400 » par la Société AMERCHOL, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société NALCO, les polymères quaternaires de vinylpyrrolidone et de vinylimidazole et leurs mélanges.

On va maintenant donner à titre d'illustration et sans aucun caractère limitatif plusieurs exemples de compositions cosmétiques.

### Exemple 1

On a préparé les compositions de shampooing suivante :

| En g | | |
|---|---|---|
| | A invention | B Comparatif |
| Lauryl éther (20E) sulfate de sodium (70 % MA) | 12 | 12 |
| Coco Bétaine (32 % m.a.) | 10 | 10 |
| Monoéthanolamide de coprah (cocamide MEA) | 0,50 | 0,50 |
| Laureth-12 | 0,25 | 0,25 |
| Acide mucique (Acide galactarique)* | 0,30 | - |
| Colorant | qs | qs |
| Parfum | 0,50 | 0,50 |
| agent conservateur | 0,40 | 0,40 |
| Hydroxyde de sodium q.s. | pH 6,7 | pH 6,7 |
| Hexylène Glycol | q.s.. | q.s |
| Eau qsp | 100 | 100 |

| | | |
|---|---|---|
| * Muciliance de Soc. Soliance | | |

On a comparé les deux formules par demi-tête sur 16 modèles. Les cheveux mouillés traités avec la composition selon l'invention sont plus lisses au toucher.
Les cheveux séchés sont plus souples et plus lisses.

### Exemple 2

On a préparé la composition de shampooing suivante :

| En g | |
|---|---|
| Lauryl éther (20E) sulfate de sodium (70 % MA) | 12 |
| Coco Bétaine (32 % m.a.) | 10 |
| Monoéthanolamide de coprah (cocamide MEA) | 0,50 |
| Laureth-12 | 0,25 |
| Acide méthylglycinediacétique sous forme de sel trisodique, en solution aqueuse à 40% (Trilon M Liquide ®-Société BASF) | **0,30** |
| Colorant | 0,01 |
| Parfum | 0,50 |
| Agent conservateur | 0,40 |
| Hydroxyde de sodium q.s. | pH 6,7 |
| Hexylène Glycol | 0,5 |
| Eau qsp | 100 |

### EXEMPLE 3

On a réalisé une composition de shampooing :

| | En g |
|---|---|
| | |
| Chlorure de Guar Hydroxypropyltrimonium | 0,05 |
| Coco-Betaine ( 32 % MA ) | 9 |
| Lauryl éther (20E) sulfate de sodium (70 % MA) | 22,2 |
| Sodium Methyl Paraben | 0,2 |
| DMDM Hydantoine | 0,25 |
| acide mucique (Acide Galactarique) | 0,3 |
| Dimethicone ( DC 200 Fluid 300000 de Dow Corning) | 2,7 |
| Mélange d'alcool cétylique et de 1-(hexadécyloxy)-2-octadécanol | 2,5 |
| Parfum | 0,5 |
| Monoisopropanolamide de coprah (cocamide MIPA) | 0,3 |
| Carbomer | 0,2 |
| Hydroxyde de sodium q.s | pH 7 |
| Eau qsp | 100 |

Les cheveux traités avec ce shampooing sont souples et disciplinés.

## Revendications

1. Utilisation d'au moins un acide carboxylique ou ses sels choisis parmi les acides de formule générale (I) ou (II) suivantes :
R¹-(CHOH)₄-CO₂X (I)
R²-N-(CH(R')COOX)₂ (II)
dans lesquelles :
• R¹ représente un groupe CH₂OH ou CO₂X,
• X représente un atome d'hydrogène ou un cation monovalent ou divalent issu d'un métal de transition, d'un métal alcalin ou alcalinoterreux, d'une amine organique ou d'un ion ammonium,
• R² représente un atome d'hydrogène ou un groupe -CH(COOX)-(CH₂)₂COOX, -CH₂CH₂OH, -CH(CH₃)-COOX ou -(CH₂)₂-N(COR")-CH₂-COOX;
• R" représente un groupe alkyle, linéaire ou ramifié, comportant de 1 à 30 atomes de carbone ou cyclique comportant de 3 à 30 atomes de carbone ;
• R' représente un groupe -CH₂-COOX lorsque R² représente un atome d'hydrogène, tandis que R' représente un atome d'hydrogène lorsque R² est différent d'un atome d'hydrogène,
comme agent de conditionnement des matières kératiniques en particulier les fibres kératiniques et plus particulièrement les cheveux dans et pour la préparation de compositions cosmétiques, notamment capillaires.

2. Utilisation selon la revendication 1, dans laquelle le cation monovalent ou divalent est choisi dans le groupe constitué par les cations de métaux alcalins, les cations de métaux alcalino-terreux et les cations divalents de métaux de transition.

3. Utilisation selon la revendication 1 ou la revendication 2, dans laquelle le ou les composés de formule (I) sont choisis dans le groupe constitué par l'acide gluconique, ses sels de métaux alcalins, ses sels de métaux alcalino-terreux, ses sels de métaux de transition, et leurs mélanges.

4. Utilisation selon l'une quelconque des revendications 1 à 3, dans laquelle le ou les composés de formule (I) sont choisis dans le groupe constitué par l'acide gluconique, le gluconate de sodium, le gluconate de potassium, le gluconate de calcium anhydre, le gluconate de calcium monohydraté, le borogluconate de calcium, le gluconate de magnésium, le gluconate de fer, le gluconate de manganèse, le gluconate de zinc et le gluconate de cuivre.

5. Utilisation selon la revendication 1 ou la revendication 2, dans laquelle le ou les composés de formule (I) sont choisis dans le groupe constitué par l'acide mucique, l'acide glucarique, l'acide mannarique, leurs sels de métaux alcalins, leurs sels de métaux alcalino-terreux, leurs sels de métaux de transition, et leurs mélanges.

6. Utilisation selon l'une quelconque des revendications précédentes, dans laquelle le ou les composés de formule (I) sont choisis parmi l'acide gluconique et l'acide mucique.

7. Utilisation selon la revendication 1 ou la revendication 2, dans laquelle le ou les composés de formule (II) sont choisis parmi l'acide méthylglycinediacétique, l'acide 2-hydroxyéthyliminodiacétique, l'acide N-lauroyl-N,N',N'-triacétique éthylènediamine, l'acide iminodisuccinique, l'acide N,N-dicarboxyméthyl L-glutamique et leurs sels correspondants, ainsi que leurs mélanges.

8. Utilisation selon l'une quelconque des revendications 1, 2 ou 7, dans laquelle le ou les composés de formule (II) est l'acide méthylglycinediacétique, éventuellement sous forme de sel.

9. Utilisation selon la revendication 1 ou la revendication 2, dans laquelle le ou les composés de formule (II) est l'acide 2-hydroxyéthyl-iminodiacétique, éventuellement sous forme de sel.

10. Utilisation selon l'une quelconque des revendications 1, 2 ou 9, dans laquelle le ou les composés de formule (II) est l'acide iminodisuccinique, éventuellement sous forme de sel.

11. Utilisation selon l'une quelconque des revendications précédentes, dans laquelle le ou les composés de formule (I) ou (II) représentent de 0,001 à 10% en poids du poids total de la composition.

12. Utilisation selon l'une quelconque des revendications précédentes, **caractérisée par le fait que** dans la composition, le ou les composés de formule (I) ou (II) représentent de 0,001 à 5% en poids du poids total de la composition.

13. Utilisation selon l'une quelconque des revendications 1 à 12, **caractérisée par le fait que** la composition peut contenir en plus des additifs cosmétiques conventionnels choisis parmi les corps gras, les solvants organiques, les silicones, les épaississants, les adoucissants, les tensio-actifs, les polymères anioniques, cationiques, non-ioniques ou amphotères, les agents anti-mousses, les protéines, des vitamines, les agents anti-chute, les agents antipelliculaires les colorants, les parfums, les conservateurs, les agents propulseurs.

14. Utilisation selon l'une quelconque des revendications 1 à 12, sous forme de shampooing, d'après-shampooing à rincer ou non, de compositions pour permanente, défrisage, coloration ou décoloration, ou encore sous forme de compositions à rincer, à appliquer avant ou après une coloration, une permanente ou un défrisage ou encore entre les deux étapes d'une permanente ou d'un défrisage.

15. Utilisation d'au moins un acide carboxylique ou ses sels choisis parmi les acides de formule générale (1) ou (II) suivantes :
R¹-(CHOH)₄-CO₂X (I)
R²-N-(CH(R')COOX)₂ (II)
dans lesquelles :
• R¹ représente un groupe CH₂OH ou CO₂X,
• X représente un atome d'hydrogène ou un cation monovalent ou divalent issu d'un métal de transition, d'un métal alcalin ou alcalinoterreux, d'une amine organique ou d'un ion ammonium,
• R² représente un atome d'hydrogène ou un groupe -CH(COOX)-(CH₂)₂COOX, -CH₂CH₂OH, -CH(CH₃)-COOX ou -(CH₂)₂-N(COR")-CH₂-COOX;
• R" représente un groupe alkyle, linéaire ou ramifié, comportant de 1 à 30 atomes de carbone ou cyclique comportant de 3 à 30 atomes de carbone ;
R' représente un groupe -CH₂-COOX lorsque R² représente un atome d'hydrogène, tandis que R' représente un atome d'hydrogène lorsque R² est différent d'un atome d'hydrogène,
pour améliorer les propriétés de démêlage, de douceur et de brillance des cheveux.
